# EUROPEAN PATENT APPLICATION

(11) **EP 4 692 331 A1**
(43) Date of publication of application: **11.02.2026**
(21) Application number: 24780820.7
(22) Date of filing: 29.03.2024
(51) Int. Cl.: C12N 5/10, C12N 5/09, C12N 5/0786, C12N 15/11, C12N 15/12, C12N 15/53, C12Q 1/02, C12Q 1/66

(54) **CELLS, METHOD FOR DETECTING PYROGENIC SUBSTANCE IN SPECIMEN, AND KIT FOR USE IN DETECTING PYROGENIC SUBSTANCE**

(30) Priority: 30.03.2023 JP 2023056676; 31.08.2023 JP 2023140762
(71) Applicant: Hoshi University, Tokyo 142-8501 (JP); FUJIFILM Wako Pure Chemical Corporation, Osaka 540-8605 (JP)
(72) Inventor: OKU Teruaki, Tokyo 142-8501 (JP); NANAO Tomohisa, Tokyo 106-8620 (JP); MARUTANI Yuki, Tokyo 106-8620 (JP)
(74) Representative: Hoffmann Eitle
(86) International application number: PCT/JP2024/013068
(87) International publication number: WO 2024/204725

(57) **Abstract**

An object of the present invention is to provide a novel cell used for detecting a pyrogenic substance by a MAT method, a method of detecting a pyrogenic substance in a specimen using the cell, and a kit for use in detecting a pyrogenic substance, containing the cell. According to the present invention, there are provided a cell which is derived from any one of a promonocyte-like cell line NOMO-1, a human-derived monocyte cell line U-937, a human-derived monoblast cell line GDM-1, a human-derived monocyte/macrophage cell line 28SC-ES, or a multiple myeloma cell line RPMI8226, in which at least one reporter gene, expression of which is controlled by a promoter inducible by NF-κB, has been introduced into the cell, and the cell has a toll-like receptor; a method for detecting a pyrogenic substance in a specimen using the cell; and a kit for use in detecting a pyrogenic substance, containing the cell.

## Description

### Technical Field

The present invention relates to a cell, a method for detecting a pyrogenic substance in a cell or a specimen, and a kit for use in detecting a pyrogenic substance.

### Background Art

In the related art, methods for detecting a pyrogenic substance in a specimen have been studied. For example, investigation of contamination by pyrogenic substances in pharmaceuticals, medical devices, and the like is essential in quality control. In addition, investigation of contamination with pyrogenic substances is also an important consideration for those that come into direct contact with the skin or mucous membranes, including those provided for human or animal consumption or those applied to human or animal.

As an evaluation method for such a pyrogenic substance, a test using a rabbit (Rabbit pyrogen test: RPT, hereinafter, RPT method) has been established. This is a method in which a rectal temperature of a rabbit is measured in advance using a temperature sensor, then the test liquid is administered into, for example, an auricular vein of the rabbit, and whether or not the test liquid contains a pyrogenic substance is determined based on the degree of temperature increase measured several hours after the administration.

In addition, as an evaluation method for the pyrogen test, a method in which a specimen is applied to a white blood cell called monocyte instead of an animal such as a rabbit, and an immune response to a stimulus by a pyrogenic substance is detected has been studied.

For example, Patent Document 1 describes gene-introduced cells for specifically detecting pyrogens in a specimen, in which the genome of the cells contains: (a) at least one gene encoding a Toll-like receptor (TLR); and (b) at least one reporter gene whose expression is controlled by a promoter inducible by NF-κB.

Patent Document 2 describes gene-introduced cells obtained by introducing a gene encoding luciferase into HL60 cells.

In addition, in Non-Patent Document 1, gene-introduced cells in which a gene encoding luciferase is introduced into MONO-MAC-6 cells (MM6 cells) are described.

### Prior Art Documents

### Patent Documents

Patent Document 1: JP2009-542236A
Patent Document 2: CN11148980A

### Non-Patent Documents

Non-Patent Document 1: Linda Palma, et al., ASSAY and Drug Development Technologies, vol. 15, No. 2, 2017, p. 64-75

### Summary of Invention

### Object to be solved by the invention

In recent years, the 3Rs (replacing experimental animals with alternative evaluation methods (Replacement), reducing the number of animals used (Reduction), and minimizing the pain and distress inflicted on animals (Refinement)) of animal welfare have been recommended, especially in Europe. Therefore, for example, since 2009, in Europe, animal experiments for cosmetics and raw materials thereof have been completely prohibited, and a law has been enacted that prohibits the sale of products for which animal experiments have been performed. Furthermore, it has been decided that the above-described RPT method will be abolished by the European Pharmacopoeia by 2026. Due to these circumstances, it has been considered to replace the test using an animal, such as the RPT method, with the Monocyte Activation Test (MAT) method.

The MAT method is a method for detecting an immune response of monocyte, which is a type of white blood cells, to stimulation by pyrogenic substances.

An object of the present invention is to provide a novel cell used for detecting a pyrogenic substance by a MAT method, a method of detecting a pyrogenic substance in a specimen using the cell, and a kit for use in detecting a pyrogenic substance, containing the cell.

### Means for solving the object

Examples of representative embodiments according to the present invention are described below.
<1> A cell which is derived from any one of a promonocyte-like cell line NOMO-1, a human-derived monoblast cell line GDM-1, a human-derived monocyte cell line U-937, a human-derived monocyte/macrophage cell line 28SC-ES, or a multiple myeloma cell line RPMI8226, in which at least one reporter gene, expression of which is controlled by a promoter inducible by NF-κB, has been introduced into the cell, and the cell has a toll-like receptor.
<2> The cell according to <1>, in which the cell is a cell derived from any one of the NOMO-1, the GDM-1, or the U-937.
<3> The cell according to <1> or <2>, in which the reporter gene encodes luciferase.
<4> The cell according to any one of <1> to <3>, in which the reporter gene encodes secretable luciferase.
<5> The cell according to any one of <1> to <4>, in which the cell is for detecting a pyrogenic substance.
<6> The cell according to any one of <1> to <5>, in which the cell has been further introduced with a toll-like receptor gene.
<7> A method for detecting a pyrogenic substance in a specimen, the method comprising:
   culturing the cell according to any one of <1> to <6> in presence of a specimen; and
   estimating an amount of a product of the reporter gene using at least one of a culture solution or the cell obtained by the culture,
   wherein a pyrogenic substance in the specimen is detected based on a result of the estimation.
<8> The method for detecting a pyrogenic substance according to <7>, in which the reporter gene encodes an enzyme, and the estimation is performed by detecting an activity of the enzyme mediated by the reporter gene.
<9> A kit for use in detecting a pyrogenic substance, the kit comprising:
   the cell according to any one of <1> to <6>.
<10> The method for detecting a pyrogenic substance according to <7>, in which the culturing is performed by mixed culture of a cell described in the following (1) and a cell described in the following (2),
   (1) a cell derived from any one of a promonocyte-like cell line NOMO-1, a human-derived monoblast cell line GDM-1, a human-derived monocyte cell line U-937, or a human-derived monocyte/macrophage cell line 28SC-ES, in which at least one reporter gene, expression of which is controlled by a promoter inducible by NF-κB, has been introduced into the cell, and the cell has a toll-like receptor,
   (2) a cell derived from a multiple myeloma cell line RPMI8226, in which at least one reporter gene, expression of which is controlled by a promoter inducible by NF-κB, has been introduced into the cell, and the cell has a toll-like receptor.
<11> The method for detecting a pyrogenic substance according to <10>, in which the cell described in the (1) is a cell derived from the promonocyte-like cell line NOMO-1.
<12> The method for detecting a pyrogenic substance according to <10> or <11>, in which reporter genes in both the cell described in the (1) and the cell described in the (2) encode an enzyme, and the estimation is performed by detecting activities of enzymes mediated by the reporter genes.
<13> The kit according to <9>, in which the cell includes a cell described in the following (1) and a cell described in the following (2),
   (1) a cell derived from any one of a promonocyte-like cell line NOMO-1, a human-derived monoblast cell line GDM-1, a human-derived monocyte/macrophage cell line 28SC-ES, or a human-derived monocyte cell line U-937, in which at least one reporter gene, expression of which is controlled by a promoter inducible by NF-κB, has been introduced into the cell, and the cell has a toll-like receptor,
   (2) a cell derived from a multiple myeloma cell line RPMI8226, in which at least one reporter gene, expression of which is controlled by a promoter inducible by NF-κB, has been introduced into the cell, and the cell has a toll-like receptor.

### Effect of the invention

According to the present invention, there are provided a novel cell used for detecting a pyrogenic substance by a MAT method, a method of detecting a pyrogenic substance in a specimen using the cell, and a kit for use in detecting a pyrogenic substance, containing the cell.

### BRIEF DESCRIPTION OF THE DRAWINGS

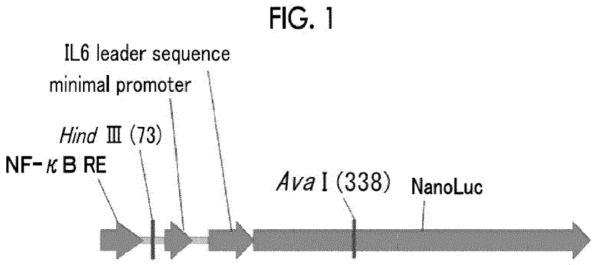

[FIG. 1] FIG. 1 is a schematic diagram showing a fully synthetic (AZENTA) luciferase gene having an NF-κB response element used in Example 1.

### Embodiments for carrying out the invention

Hereinafter, the main embodiments according to the present invention will be described. However, the present invention is not limited to the specified embodiments.

In the present specification, a numerical value range described by using "to" means a range including numerical values described before and after the preposition "to" as a lower limit value and an upper limit value, respectively.

In the present specification, unless otherwise specified, the temperature is 23°C, the atmospheric pressure is 101,325 Pa (1 atm), and the relative humidity is 50%RH.

In addition, in the present specification, a combination of preferred aspects is a more preferred aspect.

### (Cell)

The cell according to the embodiment of the present invention is a cell which is derived from any one of a promonocyte-like cell line NOMO-1, a human-derived monoblast cell line GDM-1, a human-derived monocyte cell line U-937, a human-derived monocyte/macrophage cell line 28SC-ES, or a multiple myeloma cell line RPMI8226 (hereinafter, these cell lines are also collectively referred to as "specific cell lines"), in which at least one reporter gene, expression of which is controlled by a promoter inducible by NF-κB, has been introduced into the cell, and the cell has a toll-like receptor.

As the MAT method, for example, a pyrogen test method in which a pyrogenic substance is added to peripheral blood mononuclear cells (PBMC) or a monocyte-derived cell line separated from human blood, and cytokines produced by an immune response are detected by ELISA has been studied. However, such a method has a problem that it requires skill in the ELISA operation and takes time to obtain results (for example, about 1.5 days in total).

On the other hand, a method of detecting a pyrogenic substance in a reporter assay of a transcription factor of a cytokine has also been studied. This is a method in which a pyrogenic substance is added to a cell line into which a reporter gene has been introduced, thereby producing luciferase because of an increase in transcriptional activity, and then adding a substrate to perform detection.

However, to date, no cell line having sufficient sensitivity to a pyrogenic substance has been found as compared with the above-described method using ELISA.

In addition, in the method of detecting a pyrogenic substance, it is preferable that endotoxin or a larger number of types of non-endotoxic pyrogenic substances can be detected with high sensitivity.

As a result of studies on various cell lines, the inventors of the present invention have found that cells derived from any one of a promonocyte-like cell line NOMO-1, a human-derived monoblast cell line GDM-1, a human-derived monocyte cell line U-937, a human-derived monocyte/macrophage cell line 28SC-ES, or a multiple myeloma cell line RPMI8226 are extremely useful in the detection of a pyrogenic substance, thereby completing the present invention.

### <Cell line>

The cell according to the embodiment of the present invention is a cell derived from any one of a promonocyte-like cell line NOMO-1, a human-derived monoblast cell line GDM-1, a human-derived monocyte cell line U-937, a human-derived monocyte/macrophage cell line 28SC-ES, or a multiple myeloma cell line RPMI8226.

The specific cell lines that are the cell origin of the cell according to the embodiment of the present invention are available from, for example, public storage places such as JCRB Cell Bank, Deutsche Sammlung von Mikroorganismen und Zellkulturen, and American Type Culture Collection (ATCC).

Among these, from the viewpoint of enabling the detection of a plurality of types of pyrogenic substances, the cell according to the embodiment of the present invention is preferably a cell derived from any one of the NOMO-1, the GDM-1, the U-937, or the 28SC-ES, more preferably a cell derived from any one of the NOMO-1, the GDM-1, or the U-937, still more preferably a cell derived from any one of the NOMO-1 or the GDM-1, and particularly preferably a cell derived from the NOMO-1.

In addition, the origin of the cell can also be determined in consideration of the type of the pyrogenic substance to be detected, the assumed concentration, and the like by a method such as referring to the subtypes of the toll-like receptors expressed in cells, with reference to Examples described later. It is preferable that the cell line is derived from a NOMO-1 cell line from the viewpoint that the cell line has a response ability to an agonist for a larger number of receptors among the currently known nine Toll receptors and the detection value of the pyrogenic substance obtained in Examples described later is stable.

### <Reporter gene>

The reporter gene is an exogenous gene for visualizing the expression of a certain gene in a cell.

The reporter gene according to the embodiment of the present invention may be any reporter gene whose expression is controlled by a promoter inducible by NF-xB.

For example, by introducing into cells a gene expression cassette (hereinafter, also referred to as a "reporter gene expression cassette") containing a reporter gene and an NF-κB response element located upstream of the reporter gene, the reporter gene can be placed under the control of a promoter that regulates the expression of the reporter gene and is inducible by NF-κB.

In addition, any nucleotide sequence of about 1 to 100 nucleotides may be present between each of the NF-κB response element, the minimum promoter sequence to be introduced as described later as necessary, and the reporter gene.

Here, in a case where the toll-like receptor described later is activated, it is considered that NF-κB, which localizes and binds in the cytoplasm, is released and translocates into the nucleus of the cell. Since the expression of the reporter gene is induced by this NF-κB, the activation of the toll-like receptor can be evaluated by measuring the activity (for example, the enzyme activity) of the protein encoded by the reporter gene.

The above-described reporter gene expression cassette may further have a minimal promoter sequence.

The minimal promoter sequence is preferably present downstream of the NF-κB response element and upstream of the reporter gene.

The minimal promoter is not particularly limited, and for example, a partial promoter of a promoter that is generally used for protein expression, such as an hCMV promoter or an SV40 promoter, can be used.

The reporter gene expression cassette may contain a selection marker gene such that cells into which the reporter gene expression cassette has been introduced can be selected. The selection marker gene is not particularly limited, and examples thereof include a puromycin-N-acetyltransferase gene, a hygromycin phosphotransferase gene, a neomycin phosphotransferase gene, a dihydrofolate reductase gene, and the like.

The reporter gene expression cassette may further include an enhancer sequence, a terminator sequence, a polyadenylic acid chain, and the like. These can be appropriately selected to function in the host cell.

The reporter gene is not particularly limited, and examples thereof include a gene encoding a luciferase, a chloramphenicol acetyltransferase, a β-galactosidase, a β-glucuronidase, an alkaline phosphatase, a lactamase, or a green fluorescent protein (GFP), and a gene encoding a luciferase is preferable.

The luciferase may be a cytoplasmic protein such as firefly luciferase or Renilla luciferase, or may be secretable luciferase, but from the viewpoint of reducing the influence on the measurement due to degradation in the cell, secretable luciferase is preferable.

Examples of the secretable luciferase include a luciferase sequence, which is a reporter gene sequence, to which an IL-6 secretion signal sequence or the like is added, a cypridina luciferase, a luciferase derived from a shrimp (Oplophorus gracilirostris), and a luciferase derived from a medridia (Metridia longa).

The reporter gene (reporter gene expression cassette) can be introduced into a specific cell lines by a known method such as lipofection method, liposome method, electroporation method, calcium phosphate co-precipitation method, diethylaminoethyl cellulose dextran method, or microinjection method.

In the cell according to the embodiment of the present invention, the reporter gene (reporter gene expression cassette) may be present separately from the genome or may be incorporated into the genome. Among these, it is preferable that the reporter gene is incorporated into the genome from the viewpoint of achieving stable expression of the reporter gene.

In the cell according to the embodiment of the present invention, one kind of reporter gene (reporter gene expression cassette) may be singly introduced, or two or more types thereof may be introduced.

### <Toll-like receptor (TLR)>

The cell according to the embodiment of the present invention has a Toll-like receptor (TLR).

TLR is a receptor that is present on the surface of an animal cell, and that senses various pathogens such as components of bacteria, fungi, and protozoa, and operates the innate immunity. Therefore, the cell having TLR on the surface can sense a pyrogenic substance.

It is known that there are 10 subtypes (TLR1 to TLR10) in humans and 12 subtypes (TLR1 to TLR9 and TLR11 to TLR13) in mice for TLR, but the cell according to the embodiment of the present invention preferably expresses at least one of TLR1, TLR2, TLR4, or TLR5, in terms of conforming to the NEP response determination criteria. It is more preferable that the cell expresses at least TLR4 and expresses at least one subtype of the other TLRs.

TLR4 is a receptor for lipopolysaccharide (endotoxin), which is a cell wall component of gram-negative bacteria, lipoteichoic acid in a peptidoglycan layer of gram-positive bacteria, a glycoprotein of a virus, a mite allergen, and the like. Therefore, the cell having TLR4 on the surface can sense endotoxin and the like. The cell expressing TLR4 need only co-express MD2 (lymphocyte antigen 96) for TLR4 to function.

TLR2 is a receptor that recognizes peptidoglycan, lipopeptide, a gram-positive bacterial component, and the like.

Regarding the microbial components recognized by each TLR, for example, Immunity, 34 (5), 637-650, (2011) describes the contents shown in the following table.

**[Table 1]**

| **Species** | **pathogen-associated molecular pattern** | **TLR Usage** |
|---|---|---|
| Bacteria, mycobacteria | LPS | TLR4 |
| | lipoproteins, LTA, PGN, lipoarabinomannan | TLR2/1, TLR2/6 |
| | flagell in | TLRS |
| | DNA | TLR9 |
| | RNA | TLR7 |
| Viruses | DNA | TLR9 |
| | RNA | TLR3, TLR7, TLR8 |
| | structural protein | TLR2, TLR4 |
| Fungus | zymosan, *β* -glucan | TLR2, TLR6 |
| | Mannan | TLR2, TLR4 |
| | DNA | TLR9 |
| | RNA | TLR7 |
| Parasites | tGPI-mutin (*Trypanosoma*) | TLR2 |
| | glycoinositolphospholipids (*Trypanosoma*) | TLR4 |
| | DNA | TLR9 |
| | hemozoin (*Plasmodium*) | TLR9 |
| | profilin-like molecule (*Toxoplasma gondii*) | TLR11 |

The TLR may be one that is naturally expressed by specific cell lines serving as host cells, or may be one that is expressed as a result of the introduction of a TLR gene.

It is also preferable that the cell according to the embodiment of the present invention is further introduced with a toll-like receptor gene.

By further introducing the toll-like receptor gene, for example, improvement of sensitivity to a pyrogenic substance is expected.

The introduction method is not particularly limited, and the genes can be introduced by a known transfection method.

In addition, in a case of introducing a toll-like receptor gene into the cell according to the embodiment of the present invention, a human TLR gene may be introduced, or a TLR gene of another animal species such as a mouse may be introduced.

Furthermore, in a case of introducing a toll-like receptor gene into the cell according to the embodiment of the present invention, a gene encoding an auxiliary factor such as CD14 or MD2 may be further introduced.

### <Use application>

The cell according to the embodiment of the present invention is preferably for detecting a pyrogenic substance.

In the present specification, the "pyrogenic substance" refers to a substance that causes an increase in body temperature by being incorporated into the body, and examples thereof include endotoxin (also referred to as lipopolysaccharide (LPS)) which is a pyrogenic substance derived from a gram-negative bacterium, and as a non-endotoxin pyrogenic substance (non-endotoxin pyrogen: NEP), a peptidoglycan, a pyrogenic substance derived from an exotoxin of a gram-positive bacterium, a virus, a pathogenic bacterium, a pyrogenic substance derived from a pathogenic fungus, and the like. Specific examples of the NEP include Pam3CysSerLys4 (Pam3CSK4), Heat Killed staphylogoccus aueus (HKSA), Polyinosinic-polycytidylic acid sodium salt (Poly(I:C)), Flagellin, Pam2CGDPKHPKSF (also known as Fibroblast Stimulating Lipopeptide 1: FSL-1), CL264, Resiquimod (also known as R848), Benzoazepine analog - TLR8 ligand (TL8-506), ODN2395, and the like. It is noted that such an increase in body temperature may be caused by an immune response of a sample in which the substance has been inoculated.

In a case where the cell according to the embodiment of the present invention is provided as a product or the like, the cell according to the embodiment of the present invention may be provided in a frozen state.

For example, the cells according to the embodiment of the present invention can be provided in a state of being suspended in a freezing culture medium suitable for cells and a cryoprotectant (dimethyl sulfoxide, glycerol, or the like) at about -70°C to -80°C or in a state of being frozen at about -196°C, which is the temperature of liquid nitrogen.

The freezing culture medium is not particularly limited, and may be selected according to the type of the specific cell lines. Examples thereof include a Ham's F12 medium, a MEM medium, a DMEM medium, an RPMI1640 medium, and the like. A serum such as fetal bovine serum may be added to these freezing culture media, as necessary. In addition, a commercially available freezing culture medium may be used.

The cell according to the embodiment of the present invention in a frozen state can be thawed according to a conventional method. For example, the cells according to the embodiment of the present invention frozen in the freezing culture medium may be thawed by a method such as thawing the cells at 37°C, suspending the cells in a culture medium of 10 times the amount (which may contain serum as necessary), and exchanging the culture medium after centrifugation.

In addition, the cell counting can be made unnecessary at the time of use by dispensing the cell suspension in advance to contain a certain number of cells and providing it or by dispensing the above-described freezing culture medium in advance to contain a certain number of cells and freezing the culture medium.

Furthermore, the lyophilized product may be a lyophilized product obtained by mixing two or more types of the cells according to the embodiment of the present invention. For example, a freeze-dried product of a mixture of one or more types of the cells according to the embodiment of the present invention derived from cell lines selected from NOMO-1, GDM-1, U-937, and 28 SC-ES, and the cells according to the embodiment of the present invention derived from RPMI8226 may be used. A freeze-dried product of a mixture of one or more types of the cells according to the embodiment of the present invention derived from NOMO-1 or GDM-1 and the cells according to the embodiment of the present invention derived from RPMI8226 is preferable.

### (Method for detecting pyrogenic substance)

The method for detecting a pyrogenic substance according to the embodiment of the present invention is a method including culturing the cells according to the embodiment of the present invention in the presence of a specimen, and estimating an amount of the reporter gene product using at least one of a culture solution or cells obtained by the culture, in which the pyrogenic substance in the specimen is detected based on a result of the estimation.

The term "culturing" in the present specification includes incubating the cell suspension in the presence of a specimen under warm conditions without agitation, and is not limited to cases where the cells proliferate.

### <Specimen>

The specimen is not particularly limited, and examples thereof include pharmaceuticals such as injectable solutions, solutions for injection, infusion fluids, and dialysis solutions; foods; cosmetics; biological specimens such as blood; water such as tap water and environmental water (river water, lake water, and the like); airborne substances such as airborne particulate matter, pollen, yellow sand, and airborne dust particles; house dust such as mites, mite feces, pet hair, mold, bacteria, dust, and pollen; and the like.

The liquid detection object may be used as it is or may be concentrated or diluted and used as a test substance (specimen), and the solid specimen may be used as a specimen of an extract obtained by using water, a buffer solution, or the like. In addition, in a case of detecting a pyrogenic substance attached to a medical device, or pharmaceutical or a food production equipment, water or the like obtained by washing these can be used as a specimen.

### <Method for culturing cells in presence of specimen>

In a case of culturing cells in the presence of a specimen, the cells according to the embodiment of the present invention may be placed in a container as a cell suspension having a density of, for example, a final concentration of about 5 × 10⁴ to 1 × 10⁸ cells/mL using a culture medium suitable for cells, and the specimen may be added thereto.

In addition, the cells according to the embodiment of the present invention to be cultured in the presence of a specimen may be one type of cells or may be a mixture of two or more types of the cells according to the embodiment of the present invention. For example, a mixture of one or more types of the cells according to the embodiment of the present invention derived from cell lines selected from NOMO-1, GDM-1, U-937, and 28SC-ES, and the cells according to the embodiment of the present invention derived from RPMI8226 may be used.

Here, it is considered that, by using a combination of cells of different origins, one or more types of cells selected from NOMO-1, GDM-1, U-937, and 28SC-ES, which are cells derived from monocytes or monoblasts, and cells derived from RPMI8226, which are cells of the present invention derived from multiple myeloma, a larger variety of non-endotoxin pyrogenic substances can be detected with high sensitivity. For example, this is because the cells according to the embodiment of the present invention derived from RPMI8226 have a difference such as a higher expression level of TLR9 than other cells.

In a case where a mixture of two or more types of the cells according to the embodiment of the present invention is used, a mixture of one or more types of the cells according to the embodiment of the present invention derived from NOMO-1 or GDM-1 and the cells according to the embodiment of the present invention derived from RPMI8226 is preferable.

In a case where a mixture of two or more types of the cells according to the embodiment of the present invention is used, the total amount of the mixed cells is preferably about 5 × 10⁴ to 1 × 10⁸ cells/mL.

Examples of the culture medium include IMDM, RPMI1640, α-MEM, MEM, DMEM, and the like, and a culture medium suitable for cells may be used.

In addition, the additive may include serum components such as human or bovine plasma protein fractions, fetal bovine serum, or human serum, sugars such as glucose and D-mannitol, amino acids, nucleic acid bases such as adenine, sodium hydrogen phosphate, α-tocopherol, linoleic acid, cholesterol, sodium selenite, human holotransferrin, human insulin, ethanolamine, 2-mercaptoethanol, G-CSF, GM-CSF, sodium hydrogen carbonate, methyl cellulose, and the like.

Here, to avoid the influence of the lot difference of the serum, a culture medium in which a serum component such as fetal bovine serum or human serum is not added can also be used.

In addition, as necessary, an antibiotic such as gentamicin, ampicillin, penicillin, or streptomycin; inorganic salts; and buffers such as HEPES, a phosphate buffer, or an acetate buffer may be added.

As a specific example, culture can be performed using a medium in which 2 to 10% fetal bovine serum is added, such as α-MEM medium supplemented with HPL (Human Platelet Lysate), a human platelet-derived supplement, with non-essential amino acids, or RPMI1640 medium containing hypoxanthine and/or HEPES. Sodium hydrogen carbonate, 100 units/mL of penicillin, 100 mg/mL of streptomycin, and the like may be further added to these media.

The container used for the culture is not particularly limited, and a container used in the related art, such as a 96 well plate, may be used.

The contact time between the cell according to the embodiment of the present invention and the test substance is preferably 30 minutes or more, more preferably 1 hour or more, and still more preferably 2 hours or more. In addition, the time is preferably 12 hours or less, more preferably 10 hours or less, and still more preferably 5 hours or less.

The contact time can be, for example, 3 to 6 hours. In addition, from the viewpoint of shortening the operation time, one to three hours is also one of the preferred aspects.

The cell culture product of the cell according to the embodiment of the present invention may be maintained at a temperature suitable for the survival of the cell, and the contact with the specimen may be performed at a temperature suitable for the survival of the cell. For example, the temperature is preferably 30°C to 40°C. It can be set to about 37°C among the above.

The CO₂ concentration during the culture may be a known concentration as a culture condition of the specific cell lines, and examples thereof include culturing under a condition of 5% CO₂ to 95% Air. In addition, the culture may be performed under the condition of 0% CO₂ (that is, 100% Air) by the design of the buffer and the like in the culture solution.

The amount of the specimen to be added can be determined according to the type of the substance considered to be the measurement target, the allowable concentration, the sensitivity in the cell according to the embodiment of the present invention, and the like, which are contained in the sample. In addition, the specimen may be diluted or concentrated as necessary.

For example, the Japanese Pharmacopoeia specifies that the endotoxin concentration of the injection solution should be 0.1 to 1 EU/mL or less.

Therefore, for example, in consideration of the sensitivity to endotoxin in the cell according to the embodiment of the present invention, the specimen diluted or concentrated to enable detection of at least the upper limit of the endotoxin concentration can be used.

The sensitivity of the cell according to the embodiment of the present invention to endotoxin can be confirmed in advance using a culture solution containing a known concentration of endotoxin, which is adjusted using endotoxin as a reagent.

### <Estimation of amount of reporter gene product>

The method for detecting a pyrogenic substance according to the embodiment of the present invention includes estimating the amount of the reporter gene product using at least one of the culture solution or the cells obtained by the culture.

The culture solution and the cells can be recovered by centrifugation.

**In** addition, the cells may be used as a cell lysis solution.

For example, in a case where the reporter gene encodes a secretable luciferase, the cells after culture may be dissolved together with the culture solution, and the amount of luciferase in the lysis solution may be estimated. **In** addition, as another method, the amount of luciferase in the culture solution may be estimated.

**In** addition, in a case where the reporter gene encodes luciferase having cytoplasmic localization, the cells may be collected and lysed, and the amount of luciferase in the lysis solution may be estimated.

As a method of lysing cells, a method known in the related art can be used.

Furthermore, the amount of the luciferase may be estimated in which two or more types of cells are cultured in the presence of the specimen, and then the culture solution is collected and mixed. An operation of removing cells is performed before or after the culture solutions are mixed.

**In** a case where the method of detecting a pyrogenic substance in a specimen according to the embodiment of the present invention is performed using two or more types of cells according to the embodiment of the present invention, it is desirable to culture a mixture of two or more types of cells according to the embodiment of the present invention in the presence of the specimen from the viewpoints of ease of operation, and the like.

**In** a case of performing the method for detecting a pyrogenic substance in a specimen according to the embodiment of the present invention using two or more types of the cells according to the embodiment of the present invention, for example, it is preferable to use one or more types of the cells according to the embodiment of the present invention derived from cell lines selected from NOMO-1, GDM-1, U-937, and 28SC-ES, and the cells according to the embodiment of the present invention derived from RPMI8226.

In addition, the combination of cells to be used can be determined by, for example, method such as referring to the subtypes of the toll-like receptors expressed in cells, with reference to Examples described later.

The amount of the reporter gene product may be detected by a method according to the type of the reporter.

Among these, it is preferable that the above-described reporter gene encodes an enzyme, and the above-described estimation is performed by detecting the activity of the enzyme mediated by the above-described reporter gene.

For example, in a case where the reporter gene is a luciferase gene, the luminescence of the cell by the luciferase may be visually confirmed or may be confirmed by an electron-multiplying CCD camera, or the luminescence by the luciferase in the cell lysis solution may be detected by a luminometer. In a case where the luminescence amount is measured with a luminometer, the amount of the reporter gene product can be estimated. By estimating the amount of this reporter gene product, it is possible to detect the pyrogenic substance contained in the specimen.

In addition, the amount of the reporter gene product can be estimated by a known method such as measuring the fluorescence intensity in the case of GFP or measuring the absorbance in the case of alkaline phosphatase.

Here, one of the preferred aspects of the present invention is that the above-described culture in the method for detecting a pyrogenic substance according to the embodiment of the present invention is performed by a mixed culture of the cell described in the following (1) and the cell described in the following (2).
(1) A cell derived from any one of a promonocyte-like cell line NOMO-1, a human-derived monoblast cell line GDM-1, a human-derived monocyte cell line U-937, or a human-derived monocyte/macrophage cell line 28SC-ES, in which at least one reporter gene, expression of which is controlled by a promoter inducible by NF-κB, has been introduced into the cell, and the cell has a toll-like receptor.
(2) A cell derived from a multiple myeloma cell line RPMI8226, in which at least one reporter gene, expression of which is controlled by a promoter inducible by NF-κB, has been introduced into the cell, and the cell has a toll-like receptor.

Both the (1) and the (2) are cells corresponding to the cells according to the embodiment of the present invention, and the preferred aspects are the same as the aspects described in the cells according to the embodiment of the present invention.

In the above-described aspect, the cell described in the (1) is preferably a cell derived from any one of NOMO-1, GDM-1, or U-937, more preferably a cell derived from any one of NOMO-1 or GDM-1, and still more preferably a cell derived from NOMO-1.

In addition, in the above-described aspect, it is preferable that both the cell described in the (1) and the cell described in the (2) have a reporter gene encoding an enzyme, and the estimation is performed by detecting the activity of the enzyme mediated by the reporter gene.

The reporter gene in the cell described in the (1) and the reporter gene in the cell described in the (2) may encode the same one or may encode different ones.

For example, from the viewpoints of ease of detection, and the like, the reporter gene in the cell described in (1) and the reporter gene in the cell described in (2) preferably both encode luciferase, and more preferably both encode a secretable luciferase.

Even in the above-described aspect, the measurement of the amount of the reporter gene product can be performed by the above-described method.

In the method for detecting a pyrogenic substance according to the embodiment of the present invention, it is also one of the preferred aspects of the present invention that the above-described culture includes culturing each of the cell described in the (1) and the cell described in the (2) in the presence of a specimen, and the above-described estimation is performed using a cell culture solution of the cell described in the (1) and a cell culture solution of the cell described in the (2), or the cell described in the (1) and the cell described in the (2).

In the above-described aspect, the measurement of the amount of the reporter gene product may be performed for each cell culture solution or each cell, or for example, the cell culture solutions may be mixed and measured at once.

In the above-described aspect, the preferred aspect of the cell and the preferred aspect of the measurement of the amount of the reporter gene product are as described above.

In addition, in the above-described aspect, the cell described in the (1) is preferably a cell derived from any one of NOMO-1, GDM-1, or U-937, more preferably a cell derived from any one of NOMO-1 or GDM-1, and still more preferably a cell derived from NOMO-1.

### (Method for evaluating substance that suppresses immune response induced by pyrogenic substance)

By the method for detecting a pyrogenic substance using the cell according to the embodiment of the present invention, it is possible to evaluate the effect of the specimen on inhibiting the immune response that the pyrogenic substance causes in the cell by simultaneously treating the pyrogenic substance and the specimen.

As a specific example, for example, by adding extracellular vesicles (EV, specimen) and endotoxin to the cell culture medium according to the embodiment of the present invention at the same time, performing the MAT method test using the cells according to the embodiment of the present invention, and confirming that the amount of the reporter gene product is suppressed as compared with the case where EV is not added, the immune response suppression effect by EV can be evaluated.

### (Kit)

The kit according to the embodiment of the present invention is a kit used for detecting a pyrogenic substance, which contains the cell according to the embodiment of the present invention.

The cells according to the embodiment of the present invention in the kit according to the embodiment of the present invention may be in a frozen state or in a state of a cell suspension, but are preferably in a frozen state.

The details of the aspect of the frozen state are the same as the details of the frozen aspect in the above-described cell according to the embodiment of the present invention.

The kit according to the embodiment of the present invention may include two or more types of the cells according to the embodiment of the present invention. These cells may be included in the kit in a state of being mixed with two or more types of cells, or may be included in the kit in an aspect in which one type of cell and another type of cell are included in separate containers.

For example, the kit according to the embodiment of the present invention may be a kit including a cell suspension in which cells are in a mixed state and a cell suspension in which cells are frozen in a mixed state, or may be a kit including a cell suspensions in which only one type of cells is contained or a plurality of types of cell suspensions in a frozen state.

Examples of a case where the kit according to the embodiment of the present invention includes two or more types of cells include an aspect in which the kit includes, as cells, the cell described in the (1) and the cell described in the (2).
(1) A cell derived from any one of a promonocyte-like cell line NOMO-1, a human-derived monoblast cell line GDM-1, a human-derived monocyte/macrophage cell line 28SC-ES, or a human-derived monocyte cell line U-937, in which at least one reporter gene, expression of which is controlled by a promoter inducible by NF-κB, has been introduced into the cell, and the cell has a toll-like receptor.
(2) A cell derived from a multiple myeloma cell line RPMI8226, in which at least one reporter gene, expression of which is controlled by a promoter inducible by NF-κB, has been introduced into the cell, and the cell has a toll-like receptor.

In the above-described aspect, the cell described in the (1) is preferably a cell derived from any one of NOMO-1, GDM-1, or U-937, more preferably a cell derived from any one of NOMO-1 or GDM-1, and still more preferably a cell derived from NOMO-1.

The preferred aspects of these cells are as described above.

In addition, the reporter genes in these cells may encode the same gene or may encode different genes.

The kit according to the embodiment of the present invention may further include a culture container.

The culture container is not particularly limited, and examples thereof include a dish, a petri dish, a multi-well plate, a tube, and the like.

In addition, a culture medium for cell culture may be further included.

The kit according to the embodiment of the present invention may further include a reagent for detection used for estimating the amount of the reporter gene product.

Examples of the reagent for detection include a substrate of the reporter in a case where the reporter is an enzyme, such as luciferin in a case where the reporter is luciferase, and 2-nitrophenyl-β-D-galactopyranoside (ONPG) in a case where the reporter is β-galactosidase.

Furthermore, the kit according to the embodiment of the present invention may include a manual or the like for performing the method for detecting a pyrogenic substance according to the embodiment of the present invention. The "instruction manual" means a user's manual, attached document, pamphlet (leaflet), or the like for the reagents included in the kit according to the embodiment of the present invention in which the feature, the principle, the operating procedure, the determination procedure, and the like of the method for detecting a pyrogenic substance according to the embodiment of the present invention are substantially described in sentences and/or illustrated. Specific examples thereof include materials describing the principle, operating procedure, and the like of the detection method according to the embodiment of the present invention.

In a case of using the kit according to the embodiment of the present invention, the method for detecting a pyrogenic substance according to the embodiment of the present invention can be simply, quickly, and accurately performed.

### Examples

Hereinafter, the present invention will be described in detail using examples. Materials, using amounts, proportions, treatment details, treatment content, and the like shown in the following examples can be appropriately changed without departing from the gist of the present invention. Therefore, the scope of the present invention is not limited to the specific examples described below. Unless otherwise specified, "%" is based on mass.

### Example 1. Preparation of reporter gene-introduced cell according to embodiment of present invention

### [1] Construction of reporter plasmid

A reporter plasmid was constructed by the following method based on a vector pLVSIN-CMV Pur (manufactured by Takara Bio Inc.).

The reporter plasmid was constructed by inserting the fully synthesized (AZENTA) luciferase gene having the NF-κB response element shown in FIG. 1 into the pLV-SIN-CMV-Pur vector at the site digested with the restriction enzymes ClaI (New England Biolabs, Inc.) and EcoRI (New England Biolabs, Inc.), using the In-Fusion^{™} HD Cloning Kit (manufactured by TaKaRa Bio Inc.).

The sequence information of FIG. 1 is as follows.

An insertion gene was constructed using the following sequence based on pNL 3.2.NF-κB-RE (manufactured by Promega Corporation).
NF-κB RE: NF-κB response element (NF-κB promoter sequence): (SEQ ID NO: 1)
NanoLuc: luciferase gene sequence: (SEQ ID NO: 2)

### [2] Preparation of lentivirus solution

Lenti-Pac 293Ta cells (hereinafter, referred to as "293Ta", manufactured by GeneCopoeia, Inc.) were seeded in a 10 cm dish at a concentration of 1 × 10⁶ to 1.5 × 10⁶ cells. As a culture medium, a DMEM medium containing 10% fetal bovine serum (manufactured by FUJIFILM Wako Pure Chemical Corporation) was used. After culturing in a 37°C, 5% CO₂ incubator for 48 hours, the reporter plasmid prepared in the [1] was co-introduced by lipofection using Lenti-Pac HIV Mix (manufactured by GeneCopoeia Inc.) and Endofectin (manufactured by GeneCopoeia Inc.). 293Ta into which the reporter plasmid had been introduced was cultured in a 37°C, 5% CO₂ incubator for 14 hours. After 14 hours from the start of the culture, the culture was continued for another 48 hours after the medium was exchanged with a DMEM medium containing 10% fetal bovine serum.

After the completion of the culture, the culture solution was centrifuged at 500 × G for 10 minutes to collect a supernatant (viral supernatant) containing a virus. The obtained viral supernatant was filtered through a 0.45 µm PES filter.

The titer of the number of copies was calculated for the obtained viral supernatant using a Lenti-Pac^{™} HIV qRT-PCR Titration Kit (manufactured by GeneCopoeia Inc.).

### [3] Preparation of reporter gene-introduced cell line according to embodiment of present invention

Each of the following cell lines was seeded in a 24-well plate such that the number of cells was 2 × 10⁵. The culture medium used was DMEM containing 10% heat-inactivated fetal bovine serum.
NOMO-1: JCRB Cell Bank IFO50474, National Institutes of Biomedical Innovation, Health and Nutrition
GDM-1: ATCC CRL-2627
U-937: JCRB Cell Bank JCRB9021, National Institutes of Biomedical Innovation, Health and Nutrition
28SC-ES: JCRB Cell Bank JCRB1647, National Institutes of Biomedical Innovation, Health and Nutrition
RPMI8226: JCRB Cell Bank JCRB0034, National Institutes of Biomedical Innovation, Health and Nutrition

24 hours after the seeding, the seeded cells were collected in a 1.5 mL tube, and centrifuged at 1,000 rpm for 3 minutes, and the culture medium was removed. The lentivirus solution prepared in the [2] was used, and the solution was prepared by using RPMI medium (manufactured by FUJIFILM Wako Pure Chemical Corporation) containing 10% fetal bovine serum such that the virus amount was 2.5 x 10⁸ to 1 x 10⁹ copies/well, and added to the cells. Polybrene (manufactured by Sigma-Aldrich Co. LLC) was added such that a final concentration is 5 µg/mL.

The culture plate was incubated at 37°C for 60 minutes while centrifuging under the condition of 1,200 × G.

The culture plate was cultured in a 37°C, 5% CO₂ incubator for 16 hours. After the completion of the culture, the cells were collected in a 1.5 mL tube, and centrifuged at 1,000 rpm for 3 minutes, and the culture medium was removed.

The cells were suspended in 500 µL of RPMI1640 medium containing 10% fetal bovine serum, re-seeded in a 24-well plate, and cultured for 48 hours. After the completion of the culture, the culture were expanded from a 24 well plate to a 10 cm dish. After the cells were increased to a degree of 70% confluence, puromycin was added to a 10 cm dish such that the final concentration was 5 µg/mL.

After culturing for 10 to 14 days, the living cells were subjected to expansion culture to proliferate, thereby obtaining the reporter gene-introduced cells according to the embodiment of the present invention.

### Example 2. Evaluation test of endotoxin using reporter gene-introduced cell lines according to embodiment of present invention

Each of the reporter gene-introduced cells according to the embodiment of the present invention, which had been prepared in Example 1, was seeded in a culture flask containing RPMI1640 medium (manufactured by FUJIFILM Wako Pure Chemical Corporation) containing 10% fetal bovine serum at a density of 5 × 10⁵ cells/mL, and 1α,25-dihydroxyvitamin D3 (manufactured by Sigma-Aldrich Co. LLC) was added thereto at a final concentration of 10 ng/mL, followed by culturing for 3 days. Using the cultured reporter gene-introduced cells according to the embodiment of the present invention, the responsiveness of the reporter gene-introduced cells according to the embodiment of the present invention to LPS (endotoxin) was evaluated by the following method.

The culture solution of the reporter gene-introduced cells was centrifuged at room temperature at 800 × G for 5 minutes, and the supernatant was discarded. The pellet of the reporter gene-introduced cell was suspended in a HEPES-containing RPMI1640 medium (manufactured by FUJIFILM Wako Pure Chemical Corporation) containing 2% fetal bovine serum to obtain a cell suspension. The number of cells in the cell suspension was counted using Trypan Blue.

The cell suspension was diluted with a HEPES-containing RPMI1640 medium containing 2% fetal bovine serum, and the cell density was adjusted to a density of 2 × 10⁸ cells/mL, and the diluted cell suspension was seeded in a 96-well flat plate at 50 µL per well.

As a test substance, Lipopolysaccharide (LPS: manufactured by FUJIFILM Wako Pure Chemical Corporation) was dissolved in Otsuka Distilled Water (manufactured by Otsuka Pharmaceutical Co., Ltd.) (2000 EU/mL), and the dissolved solution was prepared by 2,500-fold dilution with a HEPES-containing RPMI1640 medium containing 2% fetal bovine serum, and used as a test substance.

50 µL of the prepared test substance was added to each well of the plate seeded with the above-described cell suspension (0.4 EU/mL LPS-containing as a final concentration). Four specimens were measured.

After adding the test substance to the cell suspension, the mixture was allowed to stand in a 37°C, 5% CO₂ incubator for 3 hours (LPS treatment of the reporter gene-introduced cells). After standing, the luciferase detection reagent Nano-Glo Luciferase Assay System (manufactured by Promega Corporation) was prepared according to the reagent protocol and 100 µL thereof was added to each well. After the addition, the luminescence value (S) was measured using a luminometer (GloMax Explorer Multimode Microplate Reader, manufactured by Promega Corporation).

### (Results)

Regarding the luciferase activity, the luminescence value (N) of the control cells was set to 1, the control cells being treated in the same manner as in Example 2 except that 50 µL of the test substance was replaced with 50 µL of RPMI1640 medium containing 2% fetal bovine serum and HEPES, and the relative luminescence intensity (S/N) for each cell line was then calculated.

The results are shown in Table 2. In Table 2, cell lines exhibiting a luminescence intensity ratio of 10 or more in a case of being treated with 0.1 EU/mL LPS is indicated as A, and cell lines exhibiting a luminescence intensity ratio of less than 10 is indicated as B.

**[Table 2]**

| | Example 2 | | | | | Comparative Example 1 | | | | |
|---|---|---|---|---|---|---|---|---|---|---|
| | NOMO-1 | GDM-1 | U-937 | 28SC-ES | RPMI8226 | MM6 | HL-60 | THP-1 | Nalm-6 | SIG-M5 |
| Detection result | A | A | A | A | A | A | A | B | B | B |

### Comparative Example 1

Using the following cells, reporter gene-introduced cells were prepared by the same method as in Example 1.
MONO-MAC-6 (MM6): DSMZ ACC124
HL-60 (JCRB Cell Bank, JCRB0085, National Institutes of Biomedical Innovation, Health and Nutrition)
THP-1: JCRB Cell Bank JCRB0112.1, National Institutes of Biomedical Innovation, Health and Nutrition
Nalm-6: JCRB Cell Bank JCRB1475, National Institutes of Biomedical Innovation, Health and Nutrition
SIG-M5: Deutsche Sammlung von Mikroorganismen und Zellkulturen (DSMZ) ACC468

Using the prepared reporter gene cell, an evaluation test of endotoxin was performed using a reporter gene-introduced cell line by the same method as in Example 2.

The obtained results are shown in Table 2.

As is clear from Table 2, it was possible to detect LPS in the same manner as in a case of using the reporter gene-introduced cells derived from MM6 and HL-60 by performing the NF-xB reporter assay using each of the reporter gene-introduced cell lines according to the embodiment of the present invention derived from NOMO-1, GDM-1, U-937, 28SC-ES, and RPMI8226.

In addition, as is clear from Table 2, in a case where a reporter gene-introduced line derived from THP-1, which is a monocyte cell line, Nalm-6, which is a lymphocyte cell line, and SIG-M5, which is a monoblast cell line, was used, the luminescence intensity ratio was less than 10 in all cases.

From the above, it is found that even in a case of using a reporter gene-introduced cell derived from a cell that is not the cell according to the embodiment of the present invention, the detection of LPS may not be possible even in a case of using cells of the same type.

### Example 3. Evaluation test of Pam3CysSerLys4 (Pam3CSK4) using reporter gene-introduced cell lines according to embodiment of present invention

The test was performed by the same method as in Example 2 except that the reporter gene-introduced cell line according to the embodiment of the present invention, which was prepared in Example 1, was used and Pam3CSK4 (manufactured by InvivoGen, Inc.), which is a non-endotoxin pyrogenic substance, was used instead of LPS, and the responsiveness of the reporter gene-introduced cell line according to the embodiment of the present invention to Pam3CSK4 was evaluated. Provided that the concentration of Pam3CSK4 in the test substance was set to 10 ng/mL, and the final concentration of Pam3CSK4 was set to 5 ng/mL.

Pam3CSK4 is a synthetic tripalmitoylated lipopeptide that mimics an acylated amino terminus of a bacterial lipoprotein.

### (Results)

Regarding the luciferase activity, the luminescence value of the control cells was set to 1, the control cells being treated in the same manner as in Example 2 except that 50 µL of the test substance (including Pam3CSK4 in Example 3) was replaced with 50 µL of RPMI1640 medium containing 2% fetal bovine serum and HEPES, and the relative luminescence intensity (S/N) for each cell line was then calculated.

The results are shown in Table 3. In Table 3, cell lines exhibiting a luminescence intensity ratio of 10 or more in a case of treating with 10 ng/mL of Pam3CSK4 is indicated as A, and cell lines exhibiting a luminescence intensity ratio of less than 10 is indicated as B.

As is clear from Table 3, Pam3CSK4 could be detected by performing an NF-κB reporter assay using each of the reporter gene-introduced cell lines prepared in Example 1, which were derived from NOMO-1, U-937, 28SC-ES, and RPMI8226.

**[Table 3]**

| | Example 3 | | | | Comparative Example 2 | | | | |
|---|---|---|---|---|---|---|---|---|---|
| | NOMO-1 | U-937 | 28SC-ES | RPMI8226 | MM6 | HL-60 | THP-1 | Nalm-6 | SIG-M5 |
| Detection result | A | A | A | A | B | B | B | B | B |

### Comparative Example 2.

Using the reporter gene-introduced cells derived from MM6, HL-60, THP-1, Nalm-6, and SIG-M5, which were prepared in Comparative Example 1, the responsiveness to Pam3CSK4 was evaluated by the same method as in Example 3.

The results are shown together with Table 3.

As is clear from Table 3, it was found that Pam3CSK4 could not be detected in a case where the reporter gene-introduced cells derived from MM6, HL60, THP-1, Nalm-6, and SIG-M5 were used.

### Example 4. Evaluation test of Heat Killed Staphylococcus aureus (HKSA) using reporter gene introduced cell line

The test was performed by the same method as in Example 2 except that the reporter gene-introduced cell line according to the embodiment of the present invention, which was prepared in Example 1, was used and HKSA (manufactured by InvivoGen, Inc.), which is a non-endotoxin pyrogenic substance, was used instead of LPS, and the responsiveness of the reporter gene-introduced cell lines according to the embodiment of the present invention to HKSA was evaluated. Provided that the concentration of HKSA in the test substance was set to 1 × 10⁷ cells/mL, and the final concentration of HKSA was set to 0.5 × 10⁷ cells/mL.

### (Results)

Regarding the luciferase activity, the luminescence value of the control cells was set to 1, the control cells being treated in the same manner as in Example 2 except that 50 µL of the test substance (including HKSA in Example 4) was replaced with 50 µL of RPMI1640 medium containing 2% fetal bovine serum and HEPES, and the relative luminescence intensity (S/N) for each cell line was then calculated.

The results are shown in Table 4. In Table 4, cell lines exhibiting a luminescence intensity ratio of 10 or more in a case of treating with 1 × 10⁷ cells/mL of HKSA is indicated as A, and cell lines exhibiting a luminescence intensity ratio of less than 10 is indicated as B.

**[Table 4]**

| | Example 4 | | | | Comparative Example 3 | | | | |
|---|---|---|---|---|---|---|---|---|---|
| | NOMO-1 | GDM-1 | U-937 | 28SC-ES | MM6 | HL-60 | THP-1 | Nalm-6 | SIG-M5 |
| Detection result | A | A | A | A | A | A | B | B | B |

### Comparative Example 3.

Using the reporter gene-introduced cell lines derived from MM6, HL-60, THP-1, Nalm-6, and SIG-M5, which were prepared in Comparative Example 1, the responsiveness to HKSA (manufactured by InvivoGen, Inc.) was evaluated by the same method as in Example 4.

The results are shown together with Table 4.

As is clear from Table 4, it was possible to detect HKSA in the same manner as the reporter gene-introduced cells derived from known MM6 and HL-60 by performing the NF-κB reporter assay using the reporter gene-introduced cell lines according to the embodiment of the present invention derived from NOMO-1, GDM-1, U-937, and 28SC-ES.

In addition, as is clear from Table 4, it is found that HKSA cannot be detected in a case where the reporter gene-introduced cells derived from THP-1, Nalm-6, and SIG-M5 are used.

### Example 5. Evaluation test of Polyinosinic-polycytidylic acid sodium salt (Poly(I:C)) using reporter gene introduced cell line

The test was performed by the same method as in Example 2 except that the reporter gene-introduced cell line according to the embodiment of the present invention, which was prepared in Example 1, was used and Poly(I:C) (manufactured by Sigma-Aldrich Co. LLC), which is a non-endotoxin pyrogenic substance, was used instead of LPS, and the responsiveness of the reporter gene-introduced cell line according to the embodiment of the present invention to Poly(I:C) was evaluated. Provided that the concentration of Poly(I:C) in the test substance was set to 10 µg/mL, and the final concentration of Poly(I:C) was set to 5 µg/mL.

### (Results)

Regarding the luciferase activity, the luminescence value of the control cells was set to 1, the control cells being treated in the same manner as in Example 2 except that 50 µL of the test substance (including Poly(I:C) in Example 5) was replaced with 50 µL of RPMI1640 medium containing 2% fetal bovine serum and HEPES, and the relative luminescence intensity (S/N) for each cell line was then calculated.

The results are shown in Table 5. In Table 5, cell lines exhibiting a luminescence intensity ratio of 100 or more in a case of treating with 10 µg/mL of Poly(I:C) is indicated as A, and cell lines exhibiting a luminescence intensity ratio of less than 100 is indicated as B.

As is clear from Table 5, Poly(I:C) could be detected by performing an NF-κB reporter assay using the reporter gene-introduced cell lines according to the embodiment of the present invention derived from NOMO-1, GDM-1, U-937, 28SC-ES, and RPMI8226.

**[Table 5]**

| | Example 5 | | | | | Comparative Example 4 | | | | |
|---|---|---|---|---|---|---|---|---|---|---|
| | NOMO-1 | GDM-1 | U-937 | 28SC-ES | RPMI8226 | MM6 | HL-60 | THP-1 | Nalm-6 | SIG-M5 |
| Detection result | A | A | A | A | A | B | A | B | B | B |

### Comparative Example 4.

Using the reporter gene-introduced cell lines derived from MM6, HL-60, THP-1, Nalm-6, and SIG-M5, which were produced in Comparative Example 1, the responsiveness to Poly(I:C) was evaluated by the same method as in Example 5.

The results are shown together with Table 5.

As is clear from Table 5, it was found that Poly(I:C) could not be detected in a case where the reporter gene-introduced cell lines derived from MM6, HL-60, THP-1, Nalm-6, and SIG-M5 were used.

### Example 6. Evaluation test of Flagellin using reporter gene introduced cell line

The test was performed by the same method as in Example 2 except that the reporter gene-introduced cell line according to the embodiment of the present invention, which was prepared in Example 1, was used and Flagellin (manufactured by InvivoGen, Inc.), which is a non-endotoxin pyrogenic substance, was used instead of LPS, and the responsiveness of the reporter gene-introduced cell line according to the embodiment of the present invention to Flagellin was evaluated. Provided that the concentration of Flagellin in the test substance was set to 1 µg/mL, and the final concentration of Flagellin was set to 0.5 µg/mL.

### (Results)

Regarding the luciferase activity, the luminescence value of the control cells was set to 1, the control cells being treated in the same manner as in Example 2 except that 50 µL of the test substance (including Flagellin in Example 6) was replaced with 50 µL of RPMI1640 medium containing 2% fetal bovine serum and HEPES, and the relative luminescence intensity (S/N) for each cell line was then calculated.

The results are shown in Table 6. In Table 6, cell lines exhibiting a luminescence intensity ratio of 20 or more in a case of treating with 1 µg/mL of Flagellin is indicated as A, and cell lines exhibiting a luminescence intensity ratio of less than 20 is indicated as B.

As is clear from Table 6, Flagellin could be detected by performing an NF-κB reporter assay using the reporter gene-introduced cell lines according to the embodiment of the present invention derived from NOMO-1, GDM-1, U-937, and 28SC-ES.

**[Table 6]**

| | Example 6 | | | | Comparative Example 5 | | | | |
|---|---|---|---|---|---|---|---|---|---|
| | NOMO-1 | GDM-1 | U-937 | 28SC-ES | MM6 | HL-60 | THP-1 | Nalm-6 | SIG-M5 |
| Detection result | A | A | A | A | A | B | B | B | B |

### Comparative Example 5.

Using the reporter gene-introduced cell lines derived from MM6, HL-60, THP-1, Nalm-6, and SIG-M5, which were produced in Comparative Example 1, the responsiveness to Flagellin was evaluated by the same method as in Example 6.

The results are shown together with Table 6.

As is clear from Table 6, it is found that Flagellin can be detected in the same manner as the reporter gene-introduced cells derived from known MM6 by performing the NF-κB reporter assay using the reporter gene-introduced cell lines according to the embodiment of the present invention derived from NOMO-1, GDM-1, U-937, or 28SC-ES. In addition, it was found that Flagellin could not be detected in a case where the reporter gene-introduced cell lines derived from HL-60, THP-1, Nalm-6, and SIG-M5 were used.

### Example 7. Evaluation test of Pam2CGDPKHPSF (FSL-1) using reporter gene-introduced cell strain

The test was performed by the same method as in Example 2 except that the reporter gene-introduced cell line according to the embodiment of the present invention, which was prepared in Example 1, was used and FSL-1 (manufactured by Sigma-Aldrich Co. LLC), which is a non-endotoxin pyrogenic substance, was used instead of LPS, and the responsiveness of the reporter gene-introduced cell line according to the embodiment of the present invention to FSL-1 was evaluated. Provided that the concentration of FSL-1 in the test substance was set to 1 ng/mL, and the final concentration of FSL-1 was set to 0.5 ng/mL.

### (Results)

Regarding the luciferase activity, the luminescence value of the control cells was set to 1, the control cells being treated in the same manner as in Example 2 except that 50 µL of the test substance (including FSL-1 in Example 7) was replaced with 50 µL of RPMI1640 medium containing 2% fetal bovine serum and HEPES, and the relative luminescence intensity (S/N) for each cell line was then calculated.

The results are shown in Table 7. In Table 7, cell lines exhibiting a luminescence intensity ratio of 25 or more in a case of treating with 1 ng/mL of FSL-1 is indicated as A, and cell lines exhibiting a luminescence intensity ratio of less than 25 is indicated as B.

**[Table 7]**

| | Example 7 | | | | | Comparative Example 6 | | | |
|---|---|---|---|---|---|---|---|---|---|
| | NOMO-1 | GDM-1 | U-937 | 28SC-ES | RPMI8226 | MM6 | HL-60 | THP-1 | Nalm-6 |
| Detection result | A | A | A | A | A | A | A | B | B |

### Comparative Example 6.

Using the reporter gene-introduced cell lines derived from MM6, HL-60, THP-1, and Nalm-6, which were produced in Comparative Example 1, the responsiveness to FSL-1 was evaluated by the same method as in Example 7.

The results are shown together with Table 7.

As is clear from Table 7, it was possible to detect FSL-1 in the same manner as the reporter gene-introduced cells derived from known MM6 and HL-60 by performing the NF-κB reporter assay using the reporter gene-introduced cell lines according to the embodiment of the present invention derived from NOMO-1, GDM-1, U-937, 28SC-ES, and RPMI8226.

In addition, as is clear from Table 7, it is found that FSL-1 cannot be detected in a case where the reporter gene-introduced cell lines derived from THP-1 and Nalm-6 are used.

### Example 8. Evaluation test of CL264 using reporter gene-introduced cell line

The test was performed by the same method as in Example 2 except that each of the cells derived from NOMO-1 or RPMI8226 among the reporter gene-introduced cell lines according to the embodiment of the present invention, which was prepared in Example 1, was used and CL264 (manufactured by InvivoGen, Inc.), which is a non-endotoxin pyrogenic substance, was used instead of LPS, and the responsiveness of the reporter gene-introduced cell line according to the embodiment of the present invention to CL264 was evaluated. Provided that the concentration of CL264 in the test substance was set to 10 µg/mL, and the final concentration of CL264 was set to 5 µg/mL.

### (Results)

Regarding the luciferase activity, the luminescence value of the control cells was set to 1, the control cells being treated in the same manner as in Example 2 except that 50 µL of the test substance (including CL264 in Example 8) was replaced with 50 µL of RPMI1640 medium containing 2% fetal bovine serum and HEPES, and the relative luminescence intensity (S/N) for each cell line was then calculated.

The results are shown in Table 8. In Table 8, cell lines exhibiting a luminescence intensity ratio of 3 or more in a case of treating with 10 ng/mL of CL264 is indicated as A, and cell lines exhibiting a luminescence intensity ratio of less than 3 is indicated as B.

**[Table 8]**

| | Example 8 | | Comparative Example 7 | | | | |
|---|---|---|---|---|---|---|---|
| | NOMO-1 | RPMI8226 | MM6 | HL-60 | THP-1 | Nalm-6 | SIG-M5 |
| Detection result | A | A | B | B | B | B | B |

### Comparative Example 7.

Using the reporter gene-introduced cell lines derived from MM6, HL-60, THP-1, Nalm-6, and SIG-M5, which were produced in Comparative Example 1, the responsiveness to CL264 was evaluated by the same method as in Example 8.

The results are shown together with Table 8.

As is clear from Table 8, CL264 could be detected by performing an NF-κB reporter assay using the reporter gene-introduced cell lines according to the embodiment of the present invention derived from NOMO-1 and RPMI8226.

In addition, as is clear from Table 8, it was found that CL264 could not be detected in a case where the reporter gene-introduced cell lines derived from MM6, HL-60, THP-1, Nalm-6, and SIG-M5 were used.

### Example 9. Evaluation test of Resiquimod (R848) using reporter gene-introduced cell line

The test was performed by the same method as in Example 2 except that the reporter gene-introduced cell line according to the embodiment of the present invention, which was prepared in Example 1, was used and R848 (manufactured by InvivoGen, Inc.), which is a non-endotoxin pyrogenic substance, was used instead of LPS, and the responsiveness of the reporter gene-introduced cell line according to the embodiment of the present invention to R848 was evaluated. Provided that the concentration of R848 in the test substance was set to 1 µg/mL, and the final concentration of CL264 was set to 0.5 µg/mL.

### (Results)

Regarding the luciferase activity, the luminescence value of the control cells was set to 1, the control cells being treated in the same manner as in Example 2 except that 50 µL of the test substance (including R848 in Example 9) was replaced with 50 µL of RPMI1640 medium containing 2% fetal bovine serum and HEPES, and the relative luminescence intensity (S/N) for each cell line was then calculated.

The results are shown in Table 9. In Table 9, cell lines exhibiting a luminescence intensity ratio of 5 or more in a case of treating with 1 µg/mL of R848 is indicated as A, and cell lines exhibiting a luminescence intensity ratio of less than 5 is indicated as B.

As is clear from Table 9, R848 could be detected by performing an NF-κB reporter assay using the reporter gene-introduced cell lines according to the embodiment of the present invention derived from NOMO-1, GDM-1, U-937, 28SC-ES, and RPMI8226.

**[Table 9]**

| | Example 9 | | | | | Comparative Example 8 | | | |
|---|---|---|---|---|---|---|---|---|---|
| | NOMO-1 | GDM-1 | U-937 | 28SC-ES | RPMI8226 | MM6 | HL-60 | THP-1 | Nalm-6 |
| Detection result | A | A | A | A | A | B | A | B | B |

### Comparative Example 8.

Using the reporter gene-introduced cell lines derived from MM6, HL-60, THP-1, and Nalm-6, which were produced in Comparative Example 1, the responsiveness to R848 was evaluated by the same method as in Example 8.

The results are shown together with Table 9.

As is clear from Table 9, it is found that R848 cannot be detected in a case where the reporter gene-introduced cell lines derived from MM6, THP-1, and Nalm-6 are used.

### Example 10. Evaluation test of Benzoazepine analog - TLR8 ligand (TL8-506) using reporter gene introduced cell line

The test was performed by the same method as in Example 2 except that the reporter gene-introduced cell line according to the embodiment of the present invention, which was prepared in Example 1, was used and TL8-506 (manufactured by InvivoGen, Inc.), which is a non-endotoxin pyrogenic substance, was used instead of LPS, and the responsiveness of the reporter gene-introduced cell line according to the embodiment of the present invention to TL8-506 was evaluated. Provided that the concentration of TL8-506 in the test substance was set to 1 µg/mL, and the final concentration of TL8-506 was set to 0.5 µg/mL.

### (Results)

Regarding the luciferase activity, the luminescence value of the control cells was set to 1, the control cells being treated in the same manner as in Example 2 except that 50 µL of the test substance (including TL8-506 in Example 11) was replaced with 50 µL of RPMI1640 medium containing 2% fetal bovine serum and HEPES, and the relative luminescence intensity (S/N) for each cell line was then calculated.

The results are shown in Table 11. In Table 11, cell lines exhibiting a luminescence intensity ratio of 10 or more in a case of treating with 1 µg/mL of TL8-506 is indicated as A, and cell lines exhibiting a luminescence intensity ratio of less than 10 is indicated as B.

As is clear from Table 10, TL8-506 could be detected by performing an NF-κB reporter assay using the reporter gene-introduced cell lines according to the embodiment of the present invention derived from NOMO-1, GDM-1, U-937, and 28SC-ES.

**[Table 10]**

| | Example 10 | | | | Comparative Example 9 | | | |
|---|---|---|---|---|---|---|---|---|
| | NOMO-1 | GDM-1 | U-937 | 28SC-ES | MM6 | HL-60 | THP-1 | Nalm-6 |
| Detection result | A | A | A | A | B | A | B | B |

### Comparative Example 9.

Using the reporter gene-introduced cell lines derived from MM6, THP-1, and Nalm-6, which were produced in Comparative Example 1, the responsiveness to TL8-506 was evaluated by the same method as in Example 8.

The results are shown together with Table 9.

As is clear from Table 9, it is found that TL8-506 cannot be detected in a case where the reporter gene-introduced cell lines derived from MM6, THP-1, and Nalm-6 are used.

### <Example 11> Evaluation test of ODN2395 using reporter gene-introduced cell line

The test was performed by the same method as in Example 2 except that the reporter gene-introduced cell line according to the embodiment of the present invention, which was prepared in Example 1, was used and ODN2395 (manufactured by AdipoGen Life Sciences, Inc.), which is a non-endotoxin pyrogenic substance, was used instead of LPS, and the responsiveness of the reporter gene-introduced cell line according to the embodiment of the present invention to ODN2395 was evaluated. Provided that the concentration of R848 in the test substance was 5 µM, and the final concentration of ODN2395 was 2.5 µM.

### (Results)

Regarding the luciferase activity, the luminescence value of the control cells was set to 1, the control cells being treated in the same manner as in Example 2 except that 50 µL of the test substance (including ODN2395 in Example 10) was replaced with 50 µL of RPMI1640 medium containing 2% fetal bovine serum and HEPES, and the relative luminescence intensity (S/N) for each cell line was then calculated.

The results are shown in Table 10. In Table 10, cell lines exhibiting a luminescence intensity ratio of 1.5 or more in a case of treating with 5 µg/mL of ODN2395 is indicated as A, and cell lines exhibiting a luminescence intensity ratio of less than 1.5 is indicated as B.

As is clear from Table 10, ODN2395 could be detected by performing an NF-κB reporter assay using the reporter gene-introduced cell lines according to the embodiment of the present invention derived from NOMO-1, GDM-1, and RPMI8226.

**[Table 11]**

| | Example 11 | | | Comparative Example 10 | | | |
|---|---|---|---|---|---|---|---|
| | NOMO-1 | GDM-1 | RPMI8226 | MM6 | HL-60 | THP-1 | SIG-M5 |
| Detection result | A | A | A | B | A | B | B |

Although the data is not shown, in a case where each of Pam3CSK, 4HKSA, Poly(I:C), Flagellin, FSL-1, CL264, R848, and ODN2395 was detected by the same method as in Examples 3 to 8 using a mixture of a reporter gene-introduced cell according to the embodiment of the present invention, which is derived from NOMO-1, and a reporter gene-introduced cell derived from RPMI8226 (the mixing ratio of the number of cells: 1:1), it was possible to detect all of Pam3CSK, 4HKSA, Poly(I:C), Flagellin, FSL-1, CL264, R848, and ODN2395.

### Comparative Example 10.

Using the reporter gene-introduced cell lines derived from MM6, THP-1, and SIG-M5, which were produced in Comparative Example 1, the responsiveness to ODN2395 was evaluated by the same method as in Example 10.

The results are shown together with Table 10.

As is clear from Table 10, it is found that ODN2395 cannot be detected in a case where the reporter gene-introduced cell lines derived from MM6, THP-1, and SIG-M5 are used.
[Sequence list] International application 23F00163W1JP24013068_11.xml based on International Patent Cooperation Treaty

## Claims

1. A cell which is derived from any one of a promonocyte-like cell line NOMO-1, a human-derived monoblast cell line GDM-1, a human-derived monocyte cell line U-937, a human-derived monocyte/macrophage cell line 28SC-ES, or a multiple myeloma cell line RPMI8226,
wherein the cell has been introduced with at least one reporter gene expression of which is controlled by a promoter inducible by NF-κB, and
the cell has a toll-like receptor.

2. The cell according to claim 1,
wherein the cell is a cell derived from any one of the NOMO-1, the GDM-1, or the U-937.

3. The cell according to claim 1,
wherein the reporter gene encodes luciferase.

4. The cell according to claim 1,
wherein the reporter gene encodes secretable luciferase.

5. The cell according to claim 1,
wherein the cell is for detecting a pyrogenic substance.

6. The cell according to claim 1,
wherein the cell has been further introduced with a toll-like receptor gene.

7. A method for detecting a pyrogenic substance in a specimen, the method comprising:
culturing the cell according to any one of claims 1 to 6 in presence of a specimen; and
estimating an amount of a product of the reporter gene using at least one of a culture solution or the cell obtained by the culturing,
wherein a pyrogenic substance in the specimen is detected based on a result of the estimation.

8. The method for detecting a pyrogenic substance according to claim 7,
wherein the reporter gene encodes an enzyme, and
the estimation is performed by detecting an activity of the enzyme mediated by the reporter gene.

9. Akit for use in detecting a pyrogenic substance, the kit comprising:
the cell according to any one of claims 1 to 6.

10. The method for detecting a pyrogenic substance according to claim 7,
wherein the culturing is performed by mixed culture of a cell described in the following (1) and a cell described in the following (2),
(1) a cell derived from any one of a promonocyte-like cell line NOMO-1, a human-derived monoblast cell line GDM-1, a human-derived monocyte cell line U-937, or a human-derived monocyte/macrophage cell line 28SC-ES, in which at least one reporter gene, expression of which is controlled by a promoter inducible by NF-κB, has been introduced into the cell, and the cell has a toll-like receptor,
(2) a cell derived from a multiple myeloma cell line RPMI8226, in which at least one reporter gene, expression of which is controlled by a promoter inducible by NF-κB, has been introduced into the cell, and the cell has a toll-like receptor.

11. The method for detecting a pyrogenic substance according to claim 10,
wherein the cell described in the (1) is a cell derived from the promonocyte-like cell line NOMO-1.

12. The method for detecting a pyrogenic substance according to claim 10,
wherein reporter genes in both the cell described in the (1) and the cell described in the (2) encode an enzyme, and
the estimation is performed by detecting activities of enzymes mediated by the reporter genes.

13. The kit according to claim 9,
wherein the cell includes a cell described in the following (1) and a cell described in the following (2),
(1) a cell derived from any one of a promonocyte-like cell line NOMO-1, a human-derived monoblast cell line GDM-1, a human-derived monocyte/macrophage cell line 28SC-ES, or a human-derived monocyte cell line U-937, in which at least one reporter gene, expression of which is controlled by a promoter inducible by NF-κB, has been introduced into the cell, and the cell has a toll-like receptor,
(2) a cell derived from a multiple myeloma cell line RPMI8226, in which at least one reporter gene, expression of which is controlled by a promoter inducible by NF-κB, has been introduced into the cell, and the cell has a toll-like receptor.
